(19) Europäisches Patentamt
European Patent Office
Office européen des brevets

(11) **EP 2 243 463 A2**

(12) **EUROPÄISCHE PATENTANMELDUNG**

(43) Veröffentlichungstag:
**27.10.2010 Patentblatt 2010/43**

(51) Int Cl.:
***A61K 8/26*** *(2006.01)*     ***A61K 8/81*** *(2006.01)*
***A61K 8/92*** *(2006.01)*     ***A61Q 1/10*** *(2006.01)*

(21) Anmeldenummer: **10002847.1**

(22) Anmeldetag: **18.03.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(30) Priorität: **17.04.2009 DE 102009019006**

(71) Anmelder: **Beiersdorf AG**
**20253 Hamburg (DE)**

(72) Erfinder:
• **Klawiter, Helen**
**20255 Hamburg (DE)**
• **Diedel, Heidi**
**22083 Hamburg (DE)**
• **Viefhues, Janie**
**22589 Hamburg (DE)**
• **Schulz, Sabine**
**22159 Hamburg (DE)**

(54) **Maskara mit verbesserten rheologischen Eigenschaften**

(57) Kosmetische Zubereitung enthaltend
a) mindestes ein filmbildendes Acrylatpolymer mit einer Glasübergangstemperatur von kleiner als 20 °C,
b) ein oder mehrere anorganische Schichtsilikate,
c) ein oder mehrere organische Hydrokolloide und
d) ein oder mehrere Wachse.

EP 2 243 463 A2

**Beschreibung**

Die vorliegende Erfindung betrifft eine Maskarazubereitung mit reduzierten Faden ziehenden Eigenschaften.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Gesichts-Make-up soll der Gesichtshaut ein natürliches Aussehen verleihen, blasse Haut auffrischen, farbliche Unregelmäßigkeiten der Haut ausgleichen sowie Augen und Lippen besonders betonen.

Neben Gesichtspudern und Rouge als pulverförmigen Kosmetika sowie stiftförmigen Zubereitungen werden hierzu mehr oder weniger zähflüssige bis cremeförmige Präparate, wie Tagescremes und Creme-Make-up oder Mascara auf Emulsionsbasis verwendet.

Nachteilig am Stande der Technik ist der Umstand, dass diese fließfähigen dekorativen Zubereitungen zum "Faden ziehen" neigen. Dieser Effekt, wird im allgemeinen als "Langzügigkeit" bezeichnet und kann mit so genannten "texture-analysern" quantifiziert gemessen werden (Messverfahren siehe unten).

Der Nachteil bei Zubereitungen mit einem stark Faden ziehenden Charakter liegt u.a. darin, dass sich die Zusammensetzungen nur schwer und relativ langsam abfüllen lassen. Beim Abfüllen dieser Zubereitungen durch die Öffnung des Vorratsbehältnisses muss nach dem Befüllen des Packmittels zunächst abgewartet werden, bis der Fließstrom der Zubereitung "abreißt". Erst nach diesem "Abreißen" kann das befüllte Packmittel sauber aus der Abfüllstation entnommen und durch ein neues, leeres Vorratsbehältnis ersetzt werden. Kommt es hingegen, beispielsweise bei automatisierten Abfüllstationen, zu einem vorzeitigen Wechsel der Packmittel (d.h. bevor der Fließstrom der Zubereitung abgerissen ist), führt dies zwangsläufig dazu, dass die Zubereitung die Außenwand des Packmittels und die Transporteinrichtung der Abfüllanlage verschmutzt. Um derartige Missgeschicke zu vermeiden, muss bei "langzügigen" Zubereitungen die Abfüllgeschwindigkeit , d.h. die Anzahl zu befüllender Packmittel pro Zeiteinheit, reduziert werden. Es kann also weniger Zubereitung pro Zeit abgefüllt werden.

Auf der anderen Seite ist eine gewisse Neigung der Zubereitung zum "Faden ziehen" insbesondere bei Mascara-Zubereitungen nicht vollkommen unerwünscht, da beim Auftragen der Mascara auf die Wimpern das "Faden ziehen" nach dem Stand der Technik zu einer optischen Verlängerung der Wimpern durch die gezogenen Fäden führt.

Ein weiterer Nachteil an Zubereitungen des Standes der Technik, welche eine hohe Langzügigkeit aufweisen, liegt in dem Umstand, dass derartige Zubereitungen nach dem Trocknen (beispielsweise bei Mascara auf den Wimpern) leicht brüchige Filme bilden, die schon bei leichter mechanischer Belastung zerbröseln und nur eine geringe Abriebbeständigkeit aufweisen. Derartige Zubereitungen neigen zum Krümeln während der Tragens über die Gesamttragedauer.

Es war daher die Aufgabe der vorliegenden Erfindung, die Mängel des Standes der Technik zu beseitigen und eine leicht und schnell abfüllbare kosmetische Zubereitung (insbesondere Macara) zu entwickeln. Die Zubereitung sollte eine reduzierte Langzügigkeit aufweisen und andererseits zu schönen, optisch attraktiven dekorativen Kosmetika (insbesondere Mascara) führen. In der Ausführungsform der Mascara sollte diese den optischen Eindruck langer Wimpern verstärken.

Darüber hinaus sollten die Zubereitungen elastische Filme bilden, deren Bruch- und Abriebneigung reduziert ist.

Überraschend gelöst wird die Aufgabe durch eine kosmetische Zubereitung enthaltend

    a) mindestes ein filmbildendes Acrylatpolymer mit einer Glasübergangstemperatur von kleiner als 20 °C,
    b) ein oder mehrere anorganische Schichtsilikate,
    c) ein oder mehrere organische Hydrokolloide,
    d) ein oder mehrere Wachse

Zwar kennt der Fachmann die Offenbarungen der EP 1430868, EP 1249225, EP 0752244 und der DE 102007028498, doch konnten diese Schriften nicht den Weg zur vorliegenden Erfindung weisen.

Die erfindungsgemäßen Zubereitungen weisen erfindungsgemäß eine Langzügigkeit von $d_{max}$ <10 mm auf. Die Langzügigkeit wird dabei erfindungsgemäß mit dem folgenden Messverfahren bestimmt:

**Messung der Langzügigkeit**

Die Langzügigkeit der Zubereitung wird bei Raumtemperatur (25°C) mit dem Texturanalyser-Messgerät TA X-T2i von RHEO gemessen, ausgestattet mit einem zylindrischen Stempel aus nicht oxidierendem Metall mit einem Durchmesser von 1,2 cm. Der Stempel bewegt sich in einer senkrechten Richtung in der Zubereitung (in einem Probengefäß mit einem Durchmesser von 3,2 cm, bis zur Oberkante eingefüllt und mit einem Spatel glatt gestrichen). Es wird die Entwicklung der Kraft in Abhängigkeit der Zeit gemessen (Druck- und Streckkraft).

Der Stempel bewegt sich mit einer Geschwindigkeit von 1 mm/s und dringt in der Probe ein, bis einer Tiefe von 0,2 mm. Der Stempel wird eine Sekunde festgehalten (dies entspricht die Relaxationszeit) und wird mit 10 mm/s entfernt.

In der Relaxationsphase nimmt die (Druck-)kraft stark ab, bis sie null wird. Bei der Entfernung des Stempels wird die (Streck-)kraft negativ und nimmt dann in Richtung 0-Wert wieder zu.

Bei der Entfernung entsteht ein Zubereitungsfaden zwischen der Probenoberfläche und dem Stempel. Die Länge des Fadens steigt an, bis zu einer maximalen Länge vor Abbruch. Die Langzügigkeit oder $d_{max}$ (in mm) entspricht die maximale Länge vor Abbruch, entsprechend die vom Stempel abgelaufene Strecke während der Entfernung.

$$d_{max} \text{ (mm)} = \text{Entfernungszeit (s)} \times \text{Entfernungsgeschwindigkeit (mm/s)}$$

Die Entfernungszeit ist die Zeit, die ab der von der Zubereitung Stempelentfernung (Bildung des Zubereitungsfadens) bis zum Abbruch des Fadens vergeht.

Die Messungen der Langzügigkeit werden dreimal pro Zubereitung wiederholt.

Erfindungsgemäß weisen die erfindungsgemäßen filmbildenden Acrylatpolymere eine Glasübergangstemperatur von kleiner oder gleich 20 °C auf.

Die Glasübergangstemperatur wird erfindungsgemäß mit einem Mettler DSC 822e Gerät bestimmt. Die Probe wird dazu in einen 40μl fassenden Aluminiumtiegel eingefüllt und zweimal hintereinander im Temperaturbereich von -140°C bis +150°C bei einer Heizrate von 10°C/min vermessen. Zur Auswertung wird die zweite Aufheizkurve herangezogen.

Es ist erfindungsgemäß vorteilhaft, wenn das filmbildende Acrylatpolymer der Zubereitung in Form einer wässrigen Suspension (Latex-Dispersion) zugesetzt ist. Derartige Suspensionen verfügen erfindungsgemäß vorteilhaft über einen Feststoffanteil von 30-50 Gewichts-%, bezogen auf die Gesamtmenge der Suspension.

Es ist erfindungsgemäß vorteilhaft, wenn als filmbildendes Acrylatpolymer ein Copolymer aus zwei oder mehreren Monomeren der Gruppe Acrylat, Methacrylat, Acrylsäure und Styrol eingesetzt wird.

Es ist erfindungsgemäß bevorzugt, wenn als filmbildendes Acrylatpolymer ein Styrol/Acrylat/Ammoniummethacrylat-Copolymer (INCI: Styrene/Acrylates/Ammonium Methacrylate Copolymer) eingesetzt wird.

Die erfindungsgemäßen Styrol/Acrylat-Copolymere sind beispielsweise unter den Handelsnamen Joncryl 1532 und Joncryl ECO 2124 bei der Firma BASF Resins bzw. unter dem Handelsnamen SYNTRAN 5190, SYNTRAN 5760, SYNTRAN 5009 von Interpolymer oder aber unter der Handelsbezeichnung DOW LATEX 424 erhältlich.

Erfindungsgemäß besonders bevorzugt sind Copolymere aus Styrol, Acrylsäure, Ammonium Methacrylate (z.B. Syntran 5760).

Erfindungsgemäß vorteilhafte Ausführungsformen der vorliegenden Erfindung sind **dadurch gekennzeichnet, dass** die Konzentration an filmbildenen Acrylatpoymer in der Zubereitung von 0,1 bis 25 Gew.-% und bevorzugt von 1 bis 10 Gew.-%, jeweils bezogen auf das Gesamtgewicht der Zubereitung, beträgt.

Es ist erfindungsgemäß bevorzugt, wenn als filmbildendes Acrylatpolymer ein Copolymer aus zwei oder mehreren Mononomeren der Gruppe Acrylat, Methacrylat, Acrylsäure und Styrol eingesetzt wird, dieses Polymer in einer Gesamtkonzentration von 0,1 bis 25 Gew.-% in der Zubereitung eingesetzt wird und die Gesamtkonzentration aller filmbildenden Acrylatpolymere in der Zubereitung dabei zwischen 0,1 bis 25 Gew.-% beträgt.

Es ist erfindungsgemäß besonders bevorzugt, wenn als filmbildendes Acrylatpolymer ein Styrol/Acrylat/Ammoniummethacrylat-Copolymer (INCI: Styrene/Acrylates/Ammonium Methacrylate Copolymer) eingesetzt wird, dieses Polymer in einer Gesamtkonzentration von 1 bis 10 Gew.-% in der Zubereitung eingesetzt wird und die Gesamtkonzentration aller filmbildenden Acrylatpolymere in der Zubereitung dabei zwischen 1 bis 10 Gew.-% beträgt.

Erfindungsgemäß vorteilhafte Ausführungsformen der vorliegenden Erfindung sind **dadurch gekennzeichnet, dass** die Zubereitung zusätzlich mindestens ein Vinylpyrrolidon/Vinylacetat-Copolymer enthält.

Dabei ist es erfindungsgemäß bevorzugt, das Vinylpyrrolidon/Vinylacetat-Copolymer in einer Konzentration von 0,1 bis 25 Gew.-%, bezogen auf das Gesamtgewicht der Zubereitung einzusetzen.

Es ist erfindungsgemäß besonders bevorzugt, wenn das Vinylpyrrolidon/Vinylacetat-Copolymer in einer Konzentration von 1 bis 10 Gew.-% vorliegt.

Erfindungsgemäß besonders bevorzugte Ausführungsformen der vorliegenden Erfindung erhält man, wenn das Gewichtsverhältnis von filmbildenden Acrylatpolymer zu Vinylpyrrolidon/Vinylacetat-Copolymer zwischen 2:1 bis 1:2 liegt.

Es ist erfindungsgemäß vorteilhaft, wenn als anorganisches Schichtsilikat Magnesium-Aluminium-Schichtsilikate eingesetzt werden.

Erfindungsgemäß bevorzugt ist es, wenn der oder die anorganischen Schichtsilikate in einer Gesamtmenge von 0,01 bis 1 Gew.-%, bezogen auf das Gesamtgewicht der Zubereitung, in der Zubereitung eingesetzt werden.

Es ist erfindungsgemäß besonders bevorzugt, wenn als anorganisches Schichtsilikat Magnesium-Aluminium-Schichtsilikate eingesetzt werden, dieses in einer Gesamtkonzentration von 0,1 bis 1 Gew.-% in der Zubereitung eingesetzt wird und die Gesamtkonzentration aller anorganischen Schichtsilikate in der Zubereitung dabei 0,1 bis 1 Gew.-% beträgt.

Es ist erfindungsgemäß vorteilhaft, wenn die Gesamtkonzentration an organischen Hydrokolloiden in der Zubereitung von 0,05 bis 2 Gew.-%, bezogen auf das Gesamtgewicht der Zubereitung, beträgt.

Es ist erfindungsgemäß vorteilhaft, wenn als organisches Hydrokolloid ein oder mehrere Verbindungen gewählt aus der Gruppe der Verbindungen der vernetzen und unvernetzten Acrylat/C10-C30-Alkylacrylat-Copolymere oder Carbomere und
Ammoniumacryloyldimethyltaurat/Vinylpyrrolidon-Copolymere eingesetzt werden.

Es ist erfindungsgemäß bevorzugt, wenn als organisches Hydrokolloid vernetztes Acrylat/C10-C30-Alkylacrylat-Copolymere oder Carbomer eingesetzt wird, dieses Polymer in einer Gesamtkonzentration von 0,05 bis 1 Gew.-% in der Zubereitung eingesetzt wird und die Gesamtkonzentration aller organischen Hydrokolloide in der Zubereitung dabei zwischen 0,05 bis 2 Gew.-% beträgt.

Es ist erfindungsgemäß bevorzugt, wenn als organisches Hydrokolloid Ammoniumacryloyldimethyltaurat/Vinylpyrrolidon-Copolymer eingesetzt wird, dieses Polymer in einer Gesamtkonzentration von 0,05 bis 1 Gew.-% in der Zubereitung eingesetzt wird und die Gesamtkonzentration aller organischen Hydrokolloide in der Zubereitung dabei zwischen 0,05 bis 2 Gew.-% beträgt.

Es ist erfindungsgemäß besonders bevorzugt, wenn als organisches Hydrokolloid eine Mischung aus einer Verbindung gewählt aus der Gruppe der vernetzen und unvernetzten Acrylat/C10-C30-Alkylacrylat-Copolymere oder Carbomere und
Ammoniumacryloyldimethyltaurat/Vinylpyrrolidon-Copolymer im Gewichtsverhältnis von 1:5 bis 5:1 eingesetzt wird

Es ist erfindungsgemäß bevorzugt, wenn der oder die erfindungsgemäßen Wachse gewählt werden aus der Gruppe der Verbindungen Sonnenblumenwachs, Bienenwachs, Paraffinwachs, mikrokristallines Wachs, Carnaubawachs, Candelillawachs, Schellackwachs, Reiswachs, Polyethylenwachse

Es ist erfindungsgemäß bevorzugt, wenn die Zubereitung ein oder mehrere Wachse in einer Gesamtkonzentration von 0,1 bis 5 Gew.-%, bezogen auf das Gesamtgewicht der Zubereitung, enthält.

Dabei ist es erfindungsgemäß besonders bevorzugt, wenn die Zubereitung ein oder mehrere Wachse in einer Gesamtkonzentration von von 0,1 bis 1 Gew.-% enthält.

Erfindungsgemäß vorteilhafte Ausführungsformen der vorliegenden Erfindung sind **dadurch gekennzeichnet, dass** die Zubereitung ein oder mehrere Pigmente und/oder Farbstoffe enthält. Dabei ist es erfindungsgemäß vorteilhaft, die Pigmente und/oder Farbstoffe aus der Gruppe der folgenden Verbindungen zu wählen: Eisenoxide, Kohlenstoff (Carbon black) Glimmer, Silica, wobei die Glimmer und Silika beschichtet sein können mit beispielsweise Eisenoxid, Titandioxid bzw. Zinkoxid (z.B. Colorona Red Brown von Merck, Mica Black von Merck, Timiron Diamond Cluster MP-149 von Merck, Colorona Precious Gold von Merck).

Dabei ist der Einsatz von schwarzem Eisenoxid erfindungsgemäß bevorzugt.

Die Pigmente werden dabei üblicherweise in einer Konzentration von 0 bis 20 Gew.-%, bezogen auf das Gesamtgewicht der Zubereitung eingesetzt.

In einer erfindungsgemäß vorteilhaften Ausführungsform der vorliegenden Erfindung ist die Zubereitung frei von Alkoholen, wobei unter Alkoholen erfindungsgemäß Inhaltsstoffe verstanden werden, die als funktionelle Gruppe allein eine Alkoholfunktion aufweisen. Insbesondere ist es vorteilhaft, wenn die Zubereitung keine 1-wertigen Alkohole enthält.

Es ist erfindungsgemäß vorteilhaft, wenn die erfindungsgemäße Zubereitung ein oder mehrere hydrophile Emulgatoren enthält. Es ist in diesem Falle bevorzugt, wenn die Zubereitung Polysorbate 60, PEG-40 Hydrogenated Castor Oil oder Stearinsäure enthält.

Fakultativ kann die erfindungsgemäße Zubereitung auch weitere, für den jeweiligen Produkt-Typ typische Inhaltsstoffe, wie beispielsweise Feuchthaltemittel enthalten. Dabei ist es erfindungsgemäß vorteilhaft, folgende Verbindungen einzusetzen: Butylenglykol, Propylenglykol, Glycerin, Urea und Panthenol.

Ferner kann eine erfindungsgemäß vorteilhafte Ausführungsform der vorliegenden Erfindung ein oder mehrere Füllstoffe wie Silica, Talkum und Mica enthalten.

Die Ölphase kann beispielsweise vorteilhaft Verbindungen aus der Gruppe der polaren Öle enthalten, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Auch beliebige Abmischungen solcher Komponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Nicht zuletzt kann die Ölphase Silikonöle enthalten, beispielsweise Dimethicon und/oder Cyclomethicon.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Verdickungsmittel, die das Hautgefühl verbessern, UV-Filter oder andere übliche Bestandteile einer kosmetischen oder dermatologischen Formulierung wie Wasser, Polyole (Glycerin, Propylenglycol, Butylenglycol, Hexandiol, Octandiol, 1,3-Methylpropandiol etc.), Polymere.

Erfindungsgemäß vorteilhaft ist es, wenn die erfindungsgemäße Zubereitung als Mascara-Zubereitung verwendet wird. Wird die erfindungsgemäße Zubereitung als Mascara eingesetzt, so ist es erfindungsgemäß besonders vorteilhaft, wenn die Zubereitung mit Hilfe einer Bürste aus thermoplastischem Polyurethan aufgetragen wird. Daher ist auch ein Mascara-Produkt aus einer Bürste aus thermoplastischem Polyurethan und der erfindungsgemäßen Zubereitung erfindungsgemäß.

Erfindungsgemäß ist die Verwendung der erfindungsgemäßen kosmetischen Zubereitung zur Färbung der Haare, Wimpern, Augenbrauen, Lippen und/oder der Haut. Erfindungsgemäß ist beispielsweise der Einsatz als Mascara, als Eye Liner, als Top bzw. Base-Coat für Mascara (in double-ended Mascara), zur Augenbrauenbehandlung, als Lippenfarbe, Blush, Eyeshadow, Foundation, Konturenstift, zur Behandlung und Farbauftrag auf Haaren

**Vergleichsversuch:**

Der erfinderische Effekt konnte beispielsweise anhand des folgenden Vergleichsversuches gezeigt werden:

Mascara mit/ ohne Mg-Al-Schichtsilikat

**1. Messmethode zur Messung der Langzügigkeit:**

**Probenvorbereitung:**

Die Proben wurden in ihren Kunststoffbehältern geschlossen im Wasserbad thermostatisiert auf 25°C. Hierbei wurde im Abstand von 10 Minuten mit einem Quecksilber Glasthermometer die Probe gerührt und damit gleichzeitig festgestellt, ob die Probe homogen 25°C hat. Dieses wurde so lange durchgeführt, bis die Probe homogen 25°C hatte.

Die am Glas haftende Probenmenge wurde jeweils zurück in den Kunststoffbehälter gefüllt. Danach wurde eine entsprechende Menge mit dem Spatel entnommen und in ein für die Messung vorbereitetes Probengefäß umgefüllt.

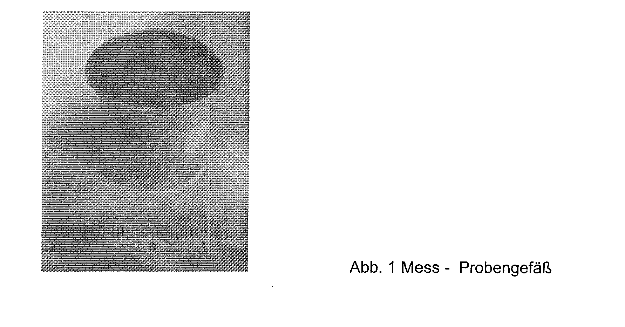

Probengefäß: siehe Abb. 1. - Höhe 25 mm, Durchmesser 27 mm, Wandstärke 1 mm, Material Aluminium.

Die Probe wurde mit der Oberkante bündig eingefüllt und mit dem Spatel glatt gestrichen. Dieses Probengefäß wurde in einen größeren Probenhalter eingeführt, so dass die Probe für die Messung weiterhin auf der Temperatur von 25°C gehalten werden konnte. Als Kontaktfläche zwischen dem Texturanalyser und der Probe wurde ein Stempel mit einem Durchmesser von 11,97 mm verwendet.

Danach erfolgte die Messung wie im Patent EP 1430868 B1 angegeben (soweit nicht explizit von dieser Beschreibung abgewichen wurde).

**Messung der Langzügigkeit**

Die Langzügigkeit der Zubereitung wird bei Raumtemperatur (25°C) mit dem Texturanalyser-Messgerät TA X-T2i von RHEO gemessen, ausgestattet mit einem zylindrischen Stempel aus nicht oxidierendes Metall mit einem Durchmesser von 1,2 cm. Der Stempel bewegt sich in einer senkrechten Richtung in der Zubereitung (in einem Probengefäß mit einem Durchmesser von 3,2 cm, bis zur Oberkante eingefüllt und mit einem Spatel glatt gestrichen). Es wird die Entwicklung der Kraft in Abhängigkeit der Zeit gemessen (Druck- und Streckkraft).

Der Stempel bewegt sich mit einer Geschwindigkeit von 1 mm/s und dringt in der Probe ein, bis einer Tiefe von 0,2 mm. Der Stempel wird eine Sekunde festgehalten (dies entspricht die Relaxationszeit) und wird mit 10 mm/s entfernt.

In der Relaxationsphase nimmt die (Druck-)kraft stark ab, bis sie null wird. Bei der Entfernung des Stempels wird die (Streck-)kraft negativ und nimmt dann in Richtung 0-Wert wieder zu.

Bei der Entfernung entsteht ein Zubereitungsfaden zwischen der Probenoberfläche und dem Stempel. Die Länge des Fadens steigt an, bis zu einer maximalen Länge vor Abbruch. Die Langzügigkeit oder dmax (in mm) entspricht die maximale Länge vor Abbruch, entsprechend die vom Stempel abgelaufene Strecke während der Entfernung.

$$d_{max} \text{ (mm)} = \text{Entfernungszeit (s)} \times \text{Entfernungsgeschwindigkeit (mm/s)}$$

Die Entfernungszeit ist die Zeit, die ab der von der Zubereitung Stempelentfernung (Bildung des Zubereitungsfadens) bis zum Abbruch des Fadens vergeht.

Die Messungen der Langzügigkeit werden dreimal pro Zubereitung wiederholt.

**Zu vermessende Proben (im Vergleich) Muster 83 und 86**

|  | Muster 83 | Muster 86 |
|---|---|---|
| Polyethoxylierte Emulgatoren | 0,5 | 0,5 |
| Organische Hydrokolloide | 1 | 1 |
| Wachs | 0,5 | 0,5 |
| Magnesium Aluminum Silicate |  | 0,1 |
| VPNA Copolymer | 3,75 | 3,75 |
| Styrene/Acrylates/Ammonium Methacrylate Copolymer | 3 | 3 |
| Pigmente | 16 | 16 |
| Konservierung | 0,5 | 0,5 |
| Polyole | 11 | 11 |
| Aqua | ad 100 | ad 100 |

**Ergebnis der Messung:**

Ergebnis:

Die Probe Nummer 86 liegt innerhalb eines Wertes von 10 mm die Probe Nummer 83 liegt außerhalb von 12 mm.

**Berechnungen:**

Aus den Messwerten ergibt sich eine Fadenlänge von:

Probe # 86    8,14 mm
Probe # 83    17,64 mm

Nach der Messmethode folgt:
$d_{max}$ (mm) = Entfernungszeit (s) x Entfernungsgeschwindigkeit (mm/s)

Probe # 86    $d_{max}$ = (1,07 - 0,25) [s] * 10 [mm s$^{-1}$] = 8,2 mm
Probe # 83    $d_{max}$ = (2,02 - 0,25) [s] * 10 [mm s$^{-1}$] = 17,7 mm

**Beispiele**

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Wasser- und Wachsphase werden auf die angegebenen Temperaturen erwärmt, vermengt und nacheinander Verdickerphase und Pigmentphase zugefügt.

|  | INCI | Beispiel 1 | Beispiel 2 | Beispiel 3 | Beispiel 4 | Beispiel 5 |
|---|---|---|---|---|---|---|
| Wasserphase 60°C | Aqua | ad 100 | ad 100 | ad 100 | ad 100 | ad 100 |
|  | Methylparaben | 0,2 | 0,2 | 0,2 | 0,2 | 0,2 |
|  | Glycerin | 6 | 6 | 6 | 6 | 6 |
|  | Panthenol | 0 | 2 | 0 | 0 | 0 |

7

(fortgesetzt)

| | INCI | Beispiel 1 | Beispiel 2 | Beispiel 3 | Beispiel 4 | Beispiel 5 |
|---|---|---|---|---|---|---|
| | Magnesium Aluminum Silicate | 0,1 | 0,05 | 0,3 | 0,3 | 0,5 |
| | Diazolidinyl Urea | 0,25 | 0,25 | 0,25 | 0,25 | 0,25 |
| | Natronlauge | 0,46 | 0,46 | 0,46 | 0,46 | 0,46 |
| | Butylene Glycol | 4,59 | 4,59 | 4,59 | 4,59 | 4,59 |
| | Polyquatemium-4 | 0,3 | 0,3 | 0,3 | 0,3 | 0,3 |
| Wachsphase 85°C | PEG-40 Hydrogenated Castor Oil | 1 | 0,4 | 0,3 | 0,3 | 0,2 |
| | Polysorbate 60 | 0,2 | 0,4 | 1 | 0,3 | 0,2 |
| | Cera Alba | 4 | 0 | 1 | 0,2 | 0,7 |
| | Jojobaöl | 0 | 2 | 0 | 0 | 0,2 |
| | Cyclomethicone | 0 | 0 | 3 | 0 | 0 |
| | Carnaubawachs | 0 | 0,5 | 0 | 0,5 | 0 |
| | Polyethylenewachs | 1 | 0 | 0 | 0 | 0 |
| Verdickerphase | Ammonium Acryloyldimethyltaurate/VP Copolymer (z.B. Aristoflex AVC von Clariant) | 1,5 | 0,2 | 0,5 | 1 | 0,4 |
| | Carbomer | 0,3 | 0,6 | 0,5 | 0,5 | 0 |
| | Carbomer | 0,2 | 0,4 | 0,3 | 0 | 0,1 |
| | Acrylat/C10-C30-Alkylacrylat-Copolymer | 0 | 0 | 0,2 | 0 | 0 |
| | VPNA Copolymer | 2 | 10 | 6 | 15 | 20 |
| | Styrene/Acrylates/AmmoniumMethacrylate Copolymer (z.B.Syntran 5760 von Interpolymer) | 12 | 20 | 3 | 7 | 10 |
| Pigmentphase | Perlglanzpigmente wie | | | | | |
| | Mica + CI 77891 (z.B. Timiron diamond Cluster MP-149 von Merck) CI 77499 + Mica + CI 77891 (z.B. Colorona mica black, Art. 117260 von Merck ) | 0 0 | 5 6 | 4 4 | 0 0 | 2 2 |
| | Eisenoxid (CI 77499) | 0 | 0 | 5 | 1 | 10 |

**Patentansprüche**

1. Kosmetische Zubereitung enthaltend

a) mindestes ein filmbildendes Acrylatpolymer mit einer Glasübergangstemperatur von kleiner als 20 °C,
b) ein oder mehrere anorganische Schichtsilikate,
c) ein oder mehrere organische Hydrokolloide
d) ein oder mehrere Wachse.

2. Kosmetische Zubereitung nach Anspruch 1, **dadurch gekennzeichnet, dass** die Zubereitung zusätzlich mindestens ein Vinylpyrrolidon/Vinylacetat-Copolymer enthält.

3. Kosmetische Zubereitung nach einem der vorhergehenden Ansprüche, **dadurch gekennzeichnet, dass** als filmbildendes Acrylatpolymer ein Copolymer aus den Mononomeren Acrylat, Methacrylat, Acrylsäure und Styrol eingesetzt wird.

4. Kosmetische Zubereitung nach einem der vorhergehenden Ansprüche, **dadurch gekennzeichnet, dass** als filmbildendes Acrylatpolymer ein Styrol/Acrylat/Ammoniummethacrylat-Copolymer (INCI: Styrene/Acrylates/Ammonium Methacrylate Copolymer) eingesetzt wird.

5. Kosmetische Zubereitung nach einem der vorhergehenden Ansprüche, **dadurch gekennzeichnet, dass** die Konzentration an filmbildenden Acrylatpolymer in der Zubereitung von 0,1 bis 25 Gew.-%, besonders bevorzugt 1 bis 10 Gew.% bezogen auf das Gesamtgewicht der Zubereitung, beträgt.

6. Kosmetische Zubereitung nach einem der vorhergehenden Ansprüche, **dadurch gekennzeichnet, dass** das filmbildende Acrylatpolymer in Kombination mit einem Vinylpyrrolidon/Vinylacetat-Copolymer im Verhältnis 2:1 bis 1:2 vorliegt.

7. Kosmetische Zubereitung nach einem der vorhergehenden Ansprüche, **dadurch gekennzeichnet, dass** als anorganisches Schichtsilikat Magnesium-Aluminium-Schichtsilikate eingesetzt werden.

8. Kosmetische Zubereitung nach einem der vorhergehenden Ansprüche, **dadurch gekennzeichnet, dass** der oder die anorganischen Schichtsilikate in einer Gesamtmenge von 0,01 bis 1 Gew.-%, bezogen auf das Gesamtgewicht der Zubereitung, in der Zubereitung eingesetzt werden.

9. Kosmetische Zubereitung nach einem der vorhergehenden Ansprüche, **dadurch gekennzeichnet, dass** als organisches Hydrokolloid ein oder mehrere Verbindungen gewählt aus der Gruppe der Verbindungen der vernetzen und unvernetzten Acrylat/C10-C30-Alkylacrylat-Copolymere oder Carbomere und Ammoniumacryloyldimethyltaurat/Vinylpyrrolidon-Copolymere eingesetzt werden.

10. Kosmetische Zubereitung nach einem der vorhergehenden Ansprüche, **dadurch gekennzeichnet, dass** die Gesamtkonzentration an organischen Hydrokolloiden in der Zubereitung von 0,05 bis 2 Gew.-%, bezogen auf das Gesamtgewicht der Zubereitung, beträgt.

11. Kosmetische Zubereitung nach einem der vorhergehenden Ansprüche, **dadurch gekennzeichnet, dass** der oder die Wachse gewählt werden aus der Gruppe der Verbindungen Sonnenblumenwachs, Bienenwachs, Paraffinwachs, mikrokristallines Wachs, Carnaubawachs, Candelillawachs, Schellackwachs, Reiswachs, Polyethylenwachse.

12. Kosmetische Zubereitung nach einem der vorhergehenden Ansprüche, **dadurch gekennzeichnet, dass** die Zubereitung ein oder mehrere Wachse in einer Gesamtkonzentration von 0,1 bis 5 Gew.%, besonders bevorzugt 0,1 bis 1 Gew. %, bezogen auf das Gesamtgewicht der Zubereitung, enthält.

13. Kosmetische Zubereitung nach einem der vorhergehenden Ansprüche, **dadurch gekennzeichnet, dass** als organisches Hydrokolloid eine Mischung aus einer Verbindung gewählt aus der Gruppe der vernetzen und unvernetzten Acrylat/C10-C30-Alkylacrylat-Copolymere oder Carbomere und Ammoniumacryloyldimethyltaurat/Vinylpyrrolidon-Copolymer im Gewichtsverhältnis von 1:5 bis 5:1 eingesetzt wird.

14. Kosmetische Zubereitung nach einem der vorhergehenden Ansprüche, **dadurch gekennzeichnet, dass** die Zubereitung Pigmente und/oder Farbstoffe enthält.

15. Verwendung einer kosmetischen Zubereitung nach einem der vorhergehenden Ansprüche zur Färbung der Haare, Wimpern, Augenbrauen, Lippen und/oder der Haut.

Abb. 1 Mess - Probengefäß

EP 2 243 463 A2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1430868 A
- EP 1249225 A
- EP 0752244 A
- DE 102007028498
- EP 1430868 B1